# FASCICULE DE BREVET EUROPEEN

(11) **EP 3 313 307 B1**
(45) Date de publication et mention de la délivrance du brevet: **13.04.2022**
(21) Numéro de dépôt: 16722244.7
(22) Date de dépôt: 13.05.2016
(51) Int. Cl.: A61B 17/64

(54) **DISPOSITIF DE FIXATION EXTERNE DYNAMIQUE D'OSTEOSYNTHESE**
DYNAMISCHE AUSSENBEFESTIGUNGSVORRICHTUNG FÜR OSTEOSYNTHESE
DYNAMIC EXTERNAL ATTACHMENT DEVICE FOR OSTEOSYNTHESIS

(30) Priorité: 29.06.2015 FR 1556079
(43) Date de publication de la demande: 02.05.2018
(73) Titulaire: GEXFIX SA, 1227 Carouge - Geneve (CH)
(72) Inventeur: PERRET, Jean-Pierre, 74300 Thiez (FR); MARLETTA, Filadelfio, 6916 Grancia (CH); VAN AAKEN, Jan, 1206 Geneve (CH); LIU, Kun, Beijing 100082 (CN)
(74) Mandataire: Papa, Elisabetta
(86) Numéro de dépôt international: PCT/EP2016/060841
(87) Numéro de publication internationale: WO 2017/001106

(56) Documents cités:
- US-A1- 2014 336 648

## Description

La présente invention concerne une paire de dispositifs de fixation externe dynamique, pour la réduction de fractures de fragments osseux par ostéosynthèse, notamment de traumatismes ostéo-articulaires des doigts de la main.

La réduction et la stabilisation d'une fracture digitale, notamment d'une fracture d'une extrémité d'une phalange, sont nécessaires afin d'autoriser une mobilisation précoce. Une mobilisation précoce reste le meilleur moyen pour lutter contre l'œdème, l'enraidissement articulaire et les adhérences.

Les fractures des phalanges sont fréquentes chez les jeunes, chez les sportifs, lors d'accident du travail ou chez les personnes âgées lors de chute.

Les traitements disponibles sont majoritairement de type, fonctionnel, orthopédique ou chirurgical, tels que par des techniques d'ostéosynthèse. Ces techniques chirurgicales d'ostéosynthèse consistent en la réduction et l'immobilisation de fragments osseux à l'aide de vis, de broches, de plaques d'ostéosynthèse ou de fixateurs externes.

Les documents US 5,976,125 et US 2014/0025075 divulguent des fixateurs externes proximal et distal, indépendants, connectés aux fragments d'os à réduire par des vis ou des broches. Les fixateurs externes proximaux et distaux sont, en outre, reliés par une tige filetée. La traction ou la distraction est réalisée par la rotation de la tige filetée. Ces dispositifs ont un encombrement conséquent. Ces fixateurs externes ne donnent pas entière satisfaction, notamment pour le confort du patient.

Le document EP 1 898 815 décrit des fixateurs externes élastiques comportant un ressort hélicoïdal coopérant avec deux corps, apte à la réduction de deux portions d'os. Chacun des deux corps comprend un alésage apte au passage d'une broche connectée à une portion d'os. Au moins une des broches traverse le ressort hélicoïdal.

Le document US 2014/0336648 décrit une paire de fixateurs externes comprenant une tige composée d'une base comprenant un alésage apte à la réception d'une première broche, d'un corps de tige localisé dans le prolongement de la base. Le corps de tige comprend, en outre, une lumière agencée selon l'axe longitudinal du corps de tige. Un ressort hélicoïdal est agencé autour du corps de tige. Une seconde broche, localisée dans la lumière du corps de tige, coopère avec le ressort.

La modulation de l'intensité de la force à exercer entre les deux portions d'os, de ces deux derniers documents, est réalisée par le pivotement du ressort. Cependant, une portion non négligeable du ressort dépasse du fixateur externe en position distale. La partie du ressort libre engendre une gêne et un inconfort dans le quotidien du patient, limitant sa liberté de mouvement et nécessitant une vigilance pour ne pas accrocher le ressort dans ses déplacements.

La présente invention propose ainsi des dispositifs de fixation externe dynamique, permettant de pallier aux inconvénients précités.

Ainsi, l'invention concerne une paire de dispositifs de fixation externe dynamique, pour la réduction de fractures de fragments osseux, les deux dispositifs sont agencés de part et d'autre d'une première portion d'os traversée par une première broche en position proximale et d'une seconde portion d'os traversée par une seconde broche en position distale. Chacun des dispositifs de fixation externe dynamique comprend un premier corps de pièce formé d'un profil longitudinal ayant un premier axe général de symétrie, le premier corps de pièce comportant au moins un alésage proximal traversant selon un second axe qui collabore avec la première broche. Le premier corps de pièce est enchâssé dans un premier ressort hélicoïdal, tandis que ce dernier est imbriqué dans un second ressort hélicoïdal, monté en collaboration avec un second corps de pièce monté en collaboration sur le premier corps de pièce. Le second corps de pièce est monté en collaboration avec la seconde broche.

Selon une alternative, le premier corps de pièce comprend une lumière agencée longitudinalement selon le premier axe général de symétrie.

Selon un mode de réalisation, le second corps de pièce est une bague solidaire et montée en bout de l'extrémité distale du second ressort, tandis que l'extrémité distale de la bague interagit avec la seconde broche agencée dans la lumière orientée parallèlement au second axe traversant l'alésage proximal du premier corps de pièce.

Selon un autre mode de réalisation, le second corps de pièce est un pivot constitué d'une tête comprenant un alésage distal interagissant avec la seconde broche, un corps collaborant avec la lumière orientée perpendiculairement au second axe traversant un alésage proximal et un élément de fermeture agencé en opposition de la tête.

Selon le mode de réalisation précédent, chacun des dispositifs selon l'invention comprend une bague solidaire et montée en bout de l'extrémité distale du second ressort, tandis que l'extrémité distale de la bague interagit avec l'extrémité proximale du pivot.

Selon un autre mode de réalisation, chacun des dispositifs comprend une bague comprenant un taraudage complémentaire à l'hélice du second ressort, et est montée en rotation sur ce dernier, tandis que l'extrémité distale du second ressort interagit avec l'extrémité proximale du pivot.

Selon une autre alternative, le second corps de pièce est une première bague comprenant un alésage distal traversant, d'axe orienté parallèlement au second axe de l'alésage proximal, la seconde broche interagissant avec l'alésage distal du second corps de pièce.

Selon un mode de réalisation, chacun des dispositifs comprend une seconde bague comprenant un taraudage complémentaire à l'hélice du second ressort, tandis que la seconde bague est montée en rotation sur le second ressort hélicoïdal, l'extrémité distale du second ressort interagissant avec l'extrémité proximale de la première bague.

Selon un autre mode de réalisation, chacun des dispositifs comprend une seconde bague solidaire de l'extrémité distale du second ressort, la seconde bague est montée en bout du second ressort, l'extrémité distale de la seconde bague interagissant avec l'extrémité proximale de la première bague.

Selon une caractéristique, le premier corps de pièce comprend une partie proximale, dans laquelle est agencé au moins un alésage proximal traversant collaborant avec la première broche, une partie centrale, et une partie distale.

Selon la caractéristique précédente, la partie proximale du premier corps de pièce a une section plus importante que celle de la partie centrale et distale, la jonction de la partie proximale et de la partie centrale forme un épaulement servant de butée en compression pour le premier ressort hélicoïdal.

Selon un mode de réalisation, l'extrémité proximale du premier ressort est engagée dans un dégagement, agencé en droit de l'épaulement, tandis que le premier ressort est bloqué en rotation par le dégagement et vient en butée contre l'épaulement.

Selon un autre mode de réalisation, la partie centrale est composée en deux parties, à savoir une partie proximale de plus grande section et une seconde partie, de plus faible section, la jonction de ces deux parties forme un second épaulement servant de butée à l'extrémité proximale du second ressort en position extrême de distraction.

Selon un autre mode de réalisation, la partie proximale du premier corps de pièce a une section cylindrique tronquée, à savoir une section comprenant deux côtés parallèles et deux côtés de forme concave en opposition.

Selon une caractéristique supplémentaire, le diamètre de l'hélice du second ressort est supérieur à celui du premier ressort.

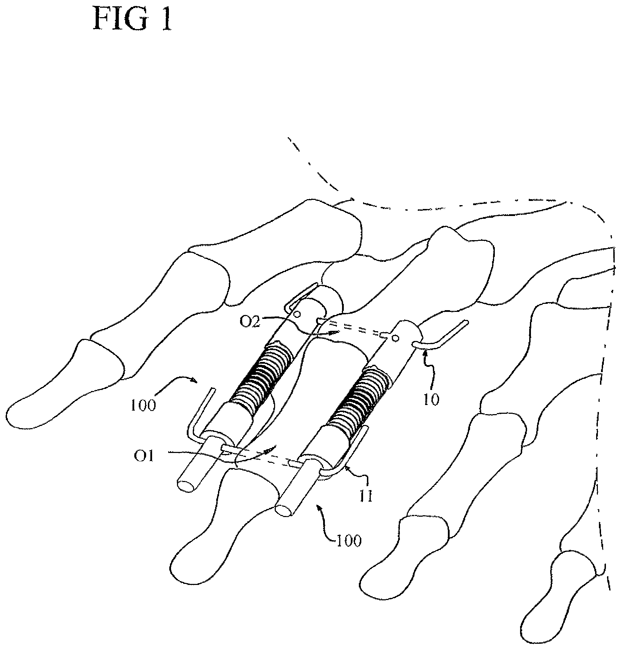

D'autres caractéristiques et avantages de l'invention se dégageront de la description qui va suivre en regard des dessins annexés qui ne sont donnés qu'à titre d'exemples non limitatifs. Seul le mode de réalisation des figures 1 à 6 appartient à l'invention telle qu'elle est revendiquée.
Les figures 1 à 6 sont des vues du dispositif de fixation externe dynamique (100), selon un mode de réalisation de l'invention.
La figure 1 est une vue en perspective d'une paire de dispositifs de fixation externe dynamique (100) en utilisation.
La figure 2 est une vue en perspective d'un dispositif de fixation externe dynamique (100).
La figure 3 est une vue de dessus selon (D) de la figure 2.
La figure 4 est une vue de face selon (F) de la figure 2.
La figure 5 est une vue partielle, en perspective, du dispositif de fixation externe dynamique (100), illustrant le premier corps de pièce (1) solidaire du second ressort (13).
La figure 6 est une vue partielle, en perspective, du dispositif de fixation externe dynamique (100), illustrant le second corps de pièce (2).
La figure 7 est une vue en perspective d'un dispositif de fixation externe dynamique (100) selon un mode de réalisation qui n'appartient pas à l'invention telle qu'elle est revendiquée.
Les figures 8 à 10 sont des vues d'un dispositif de fixation externe dynamique (100) selon un exemple qui n'appartient pas à l'invention telle qu'elle est revendiquée.

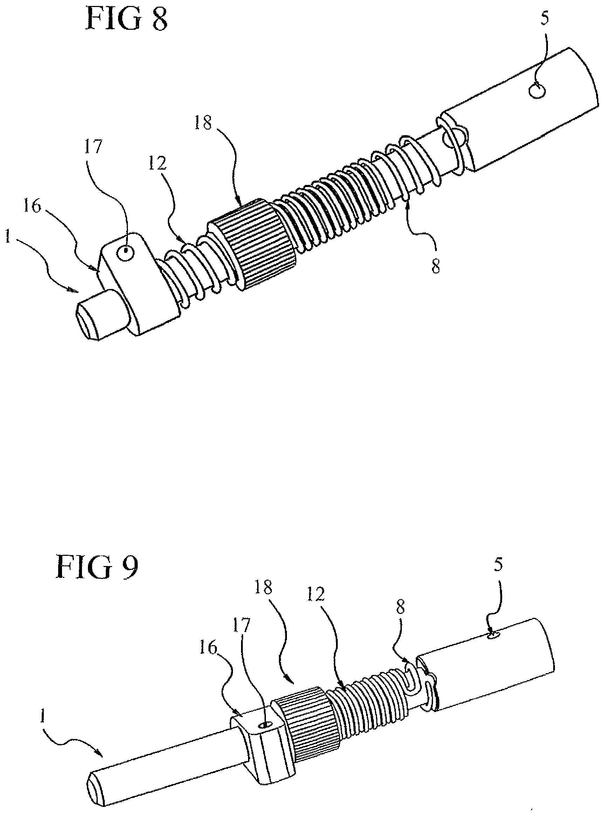

La figure 8 est une vue en perspective d'un dispositif de fixation externe dynamique (100) en position extrême de traction.
La figure 9 est une vue en perspective d'un dispositif de fixation externe dynamique (100) en position extrême de distraction.

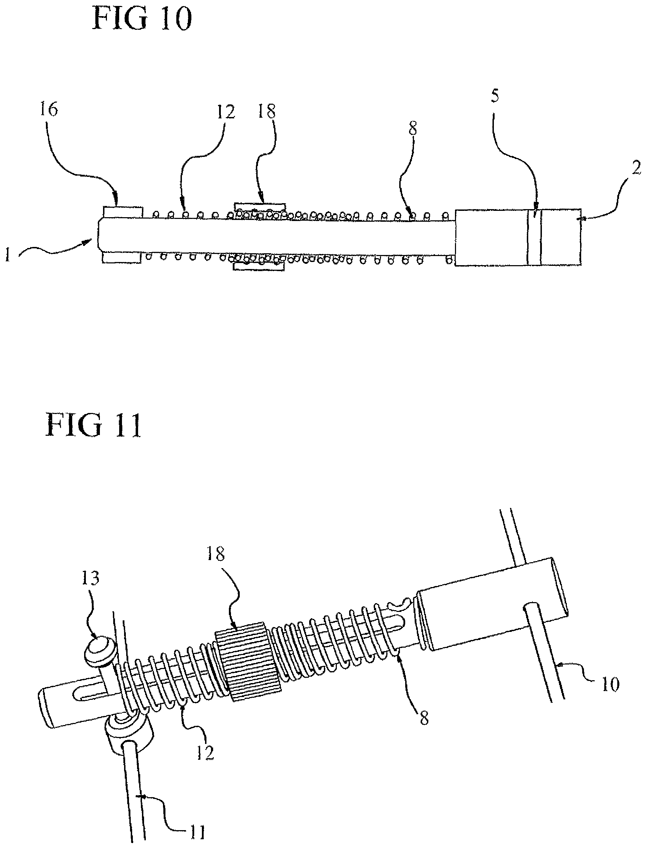

La figure 10 est une vue en coupe de la figure 8.

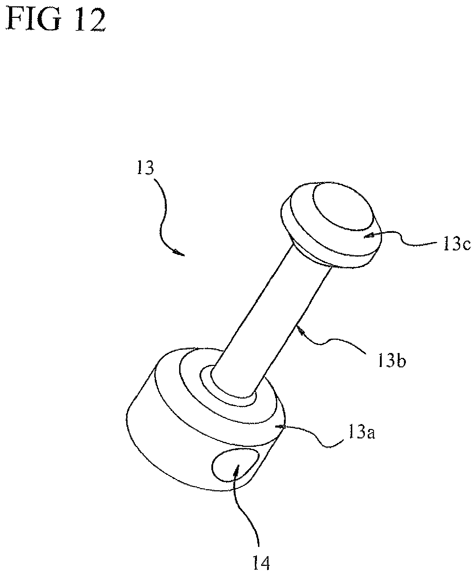

Les figures 11 et 12 sont des vues du dispositif de fixation externe dynamique (100), selon un autre exemple qui n'appartient pas à l'invention telle qu'elle est revendiquée.
La figure 11 est une vue en perspective du dispositif de fixation externe dynamique (100).
La figure 12 est une vue en perspective d'un pivot (14).

Ainsi, l'invention se présente, telle qu'illustrée à la figure 1, sous la forme d'une paire de dispositifs de fixation externe dynamique (100), agencée de part et d'autre de deux parties d'os à consolider, à savoir une première partie d'os (O1) localisée en partie proximale et une seconde partie d'os (02) localisée en partie distale. La paire de dispositifs de fixation externe dynamique (100) est reliée par deux broches (10, 11), à savoir, une première broche (10) en position proximale, et une seconde broche (11) en position distale, qui traversent de part en part les parties d'os (O1, O2) à consolider.

Ajoutons qu'un dispositif de fixation externe dynamique (100) selon l'invention comprend un premier corps de pièce (1) ayant un profil longitudinal, d'axe général (X, X'), monté en collaboration avec un second corps de pièce. Chacun des deux corps de pièce est monté en collaboration avec un ressort hélicoïdal (8, 12). Les ressorts hélicoïdaux (8, 12) sont montés imbriqués en opposition, leurs hélices respectives étant assemblées l'une avec l'autre.

Le second ressort (12) est imbriqué en rotation dans le premier ressort (8). Le second ressort (12) est soumis à des mouvements de rotation, de compression et de détente, tandis que le premier ressort (8) est uniquement soumis à des mouvements de compression et de détente.

Selon des modes de réalisation, le second ressort (12) a avantageusement un diamètre d'hélice supérieur à celui du premier ressort (8). Cette caractéristique permet l'entraînement en rotation du second ressort (12) par une bague (18) montée en rotation sur ce dernier par un taraudage approprié, tel qu'explicité plus en détail dans la suite de la description.

Selon d'autres modes de réalisation, les deux ressorts (8, 12) ont un même diamètre d'hélice, avantageusement lorsque que le second corps de pièce, à savoir une bague (18) est montée, solidaire, en bout, de l'extrémité distale du second ressort (8).

Précisons que le réglage de la traction ou de la distraction des deux parties d'os (O1, O2), via les broches (10, 11), est réalisé par la rotation du second ressort (12). Ce mouvement de rotation du second ressort (12), est avantageusement engendré soit seul, soit par la rotation du second corps de pièce soit par la rotation d'une bague (18). En distraction, l'un des deux ou les deux ressorts (8, 12) subit/subissent avantageusement un mouvement de compression, tandis qu'en traction, ces ou l'un de ces derniers subissent/subit avantageusement une détente.

Notons que le premier corps de pièce (1), comprend une partie proximale (2), dans laquelle est agencé au moins un alésage proximal (5) traversant d'axe (Y, Y'), une partie centrale (3), et une partie distale (4).

Selon un mode de réalisation, le premier corps de pièce (1) comprend un alésage proximal (5).

Selon des modes de réalisations, le premier corps de pièce (1) comprend deux alésages proximaux (5), plus préférentiellement trois alésages proximaux (5). Ces derniers sont avantageusement alignés selon un axe parallèle à l'axe (X, X').

Selon des modes de réalisation, au moins la partie distale (4) comprend une lumière (6) longitudinale le long de l'axe (X, X'). Le plan traversant la lumière (6) est agencé parallèlement à l'axe (Y, Y') traversant un alésage proximal (5), mais il pourrait en être autrement, le plan traversant la lumière (6) est agencé perpendiculairement à l'axe (Y, Y') traversant l'alésage proximal (5).

Précisons que la partie proximale (2) du premier corps de pièce (1) a une section plus importante que celle de la partie centrale (3) et distale (4). La jonction de la partie proximale (2) et de la partie centrale (3) forme un premier épaulement (7) servant de butée en compression pour le premier ressort hélicoïdal (8) monté au moins le long de la partie centrale (3).

Selon une caractéristique, l'extrémité proximale du premier ressort (8) est engagée dans un dégagement (9), agencé en droit du premier épaulement (7). Le premier ressort (8) est ainsi bloqué en rotation par le dégagement (9) et vient en butée contre le premier épaulement (7) lors de mouvement de compression.

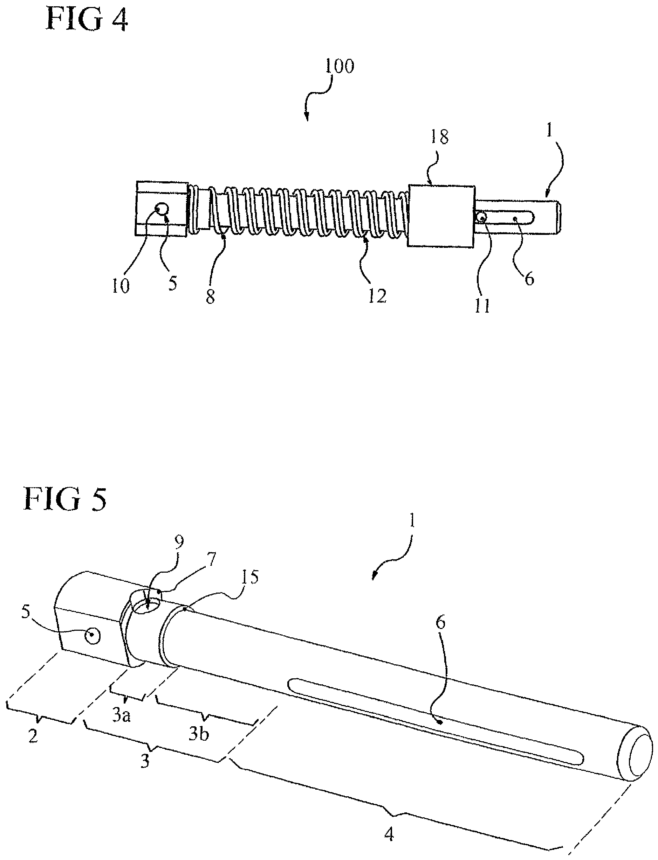

Selon une autre caractéristique, la partie proximale (2) a une section cylindrique tronquée. Cette section, selon un mode de réalisation, telle qu'illustrée à la figure 5, a deux côtés parallèles et deux côtés de forme concave en opposition. Cette configuration particulière permet un encombrement minimal lorsqu'un ou les deux dispositif(s) de fixation externe (100) est/sont placé(s), par exemple, entre deux doigts.

Selon une caractéristique supplémentaire, la partie centrale (3) est composée en deux parties, à savoir une première partie (3a) de plus grande section et une seconde partie (3b), de plus faible section. La jonction de ces deux dernières parties (3a, 3b), de la partie centrale (3), forme un second épaulement (15) servant de butée au second ressort (12), en position extrême de distraction.

Selon des modes de réalisation, la seconde partie (3b) a avantageusement une section pleine afin de limiter la taille de la lumière (6) agencée au moins en partie distale (4) et ainsi de limiter le déplacement de la seconde broche (11), insérée dans cette dernière.

Ajoutons que le second corps de pièce a avantageusement une section cylindrique et un diamètre intérieur égal ou sensiblement supérieur au diamètre extérieur de la partie distale (4) du premier corps de pièce (1), mais il pourrait en être autrement, le diamètre intérieur du second corps de pièce est égal ou sensiblement supérieur au diamètre du second ressort (12).

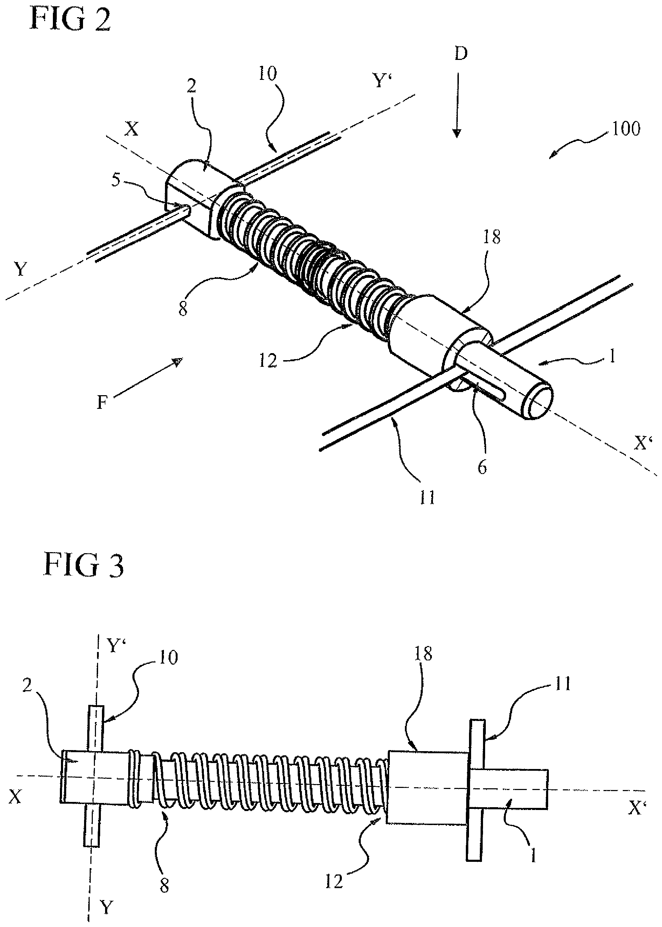

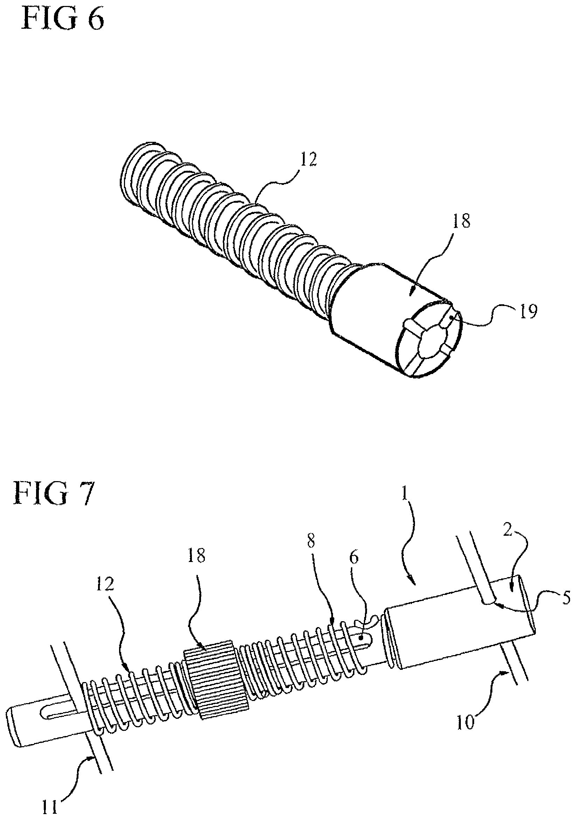

Selon un mode de réalisation, tel qu'illustré aux figures 2 à 6, le premier corps de pièce (1), tel qu'illustré plus en détail à la figure 5, comprend une lumière longitudinale agencée parallèlement à l'axe (Y, Y') traversant un alésage proximal (5). La seconde broche (11) en partie distale (4) est agencée dans la lumière (6). Le second corps de pièce, tel qu'illustré plus en détail à la figure 6, est une bague (18) solidaire de l'extrémité distale du second ressort hélicoïdal (12). L'ensemble constitué par la bague (18) et le second ressort (12) est monté en rotation sur le premier corps de pièce (1). La partie distale du second corps de pièce vient en butée contre la seconde broche (11). Le second corps de pièce est ainsi localisé entre les deux broches (10, 11), plus précisément borné par la seconde broche (11) et la partie proximale (2) du premier corps de pièce (1), plus précisément borné par la partie centrale (3) de ce dernier. Des rainures (19) sont agencées au niveau de l'extrémité distale du second corps de pièce afin de faciliter l'engagement et le maintien de ce dernier contre la seconde broche (11).

Selon une alternative qui n'appartient pas à l'invention telle qu'elle est revendiquée, telle qu'illustrée à la figure 7, le premier corps de pièce comprend un lumière (6) agencée parallèlement à l'axe (Y, Y') traversant un alésage proximal (5), tandis que le second corps de pièce est une bague (18) montée en rotation sur le second ressort (12). Le second corps de pièce comprend dans sa paroi intérieure un taraudage complémentaire à l'hélice du second ressort (12). Le second ressort (12) est en outre monté en rotation sur le premier corps de pièce (1). La partie distale du second ressort (12) vient en butée contre la seconde broche (11). La seconde broche (11) en partie distale (4) est agencée dans la lumière (6).

Selon un mode d'exécution, tel qu'illustré aux figures 8 à 10, le second corps de pièce est une première bague (16) comprenant un alésage distal (17) d'axe orienté parallèlement à l'axe (Y, Y') de l'alésage proximal (5), la seconde broche (11) étant agencée dans l'alésage (17). Le second ressort (12) est monté en collaboration avec la première bague (16), à savoir, l'extrémité distale du second ressort (12) vient en butée contre la partie proximale de la première bague (16), tandis qu'une seconde bague (18) est montée en rotation sur le second ressort (12). La seconde bague (18) comprend dans sa paroi intérieure un taraudage complémentaire à l'hélice du second ressort (12).

Selon une alternative du mode d'exécution précédent, la seconde bague (18) n'est pas montée en rotation sur le second ressort (12), mais en bout de ce dernier.

Selon une autre alternative du mode d'exécution précédent, le dispositif de fixation externe (100) ne comprend pas de seconde bague (18).

Selon un autre mode d'exécution, qui n'appartient pas à l'invention telle qu'elle est revendiquée, tel qu'illustré à la figure 11, la lumière (6) est agencée perpendiculairement à l'axe (Y, Y') traversant un alésage proximal (5). Le second corps de pièce est un pivot (13), comprenant un alésage distal (14). Le pivot (13) est inséré dans la lumière (6). Ce pivot (13) est constitué d'une tête (13a) comprenant un alésage distal (14), un corps (13b) collaborant avec la lumière (6) et un élément de fermeture (13c). Il est entendu que la seconde broche (11) est insérée dans l'alésage distal (14) de la tête (13a) du pivot (13), afin d'être agencée parallèlement à la première broche (10). Une bague (18), telle qu'illustrée plus en détail à la figure 6, est solidaire de l'extrémité distale du second ressort hélicoïdal (12). L'ensemble constitué par la bague (18) et le second ressort (12) est monté en rotation sur le premier corps de pièce (1). L'extrémité distale de la bague (18) vient en butée contre la partie proximale du pivot (13).

Selon une alternative du mode d'exécution précédent, la seconde bague (18) n'est pas montée en bout sur le second ressort (12), mais en rotation sur ce dernier.

Selon une autre alternative du mode d'exécution précédent, le dispositif de fixation externe (100) ne comprend pas de seconde bague (18).

## Revendications

1. Paire de dispositifs de fixation externe dynamique (100) pour la réduction de fractures de fragments osseux, lesdits dispositifs (100) étant configurés pour être agencés de part et d'autre d'une première portion d'os (O1) traversée par une première broche (10) en position proximale et d'une seconde portion d'os (02) traversée par une seconde broche (11) en position distale, tandis que chacun des dispositifs de fixation externe dynamique (100) comprend un premier corps de pièce (1) et un second corps de pièce, le premier corps de pièce (1) ayant un profil longitudinal s'étendant selon un premier axe (X, X'), comportant un alésage proximal (5) traversant selon un second axe (Y, Y') dans lequel la première broche (10) est configurée pour être insérée, ledit premier corps de pièce (1) étant enchâssé dans un premier ressort hélicoïdal (8), **caractérisé en ce que** ledit premier ressort hélicoïdal (8) est imbriqué dans un second ressort hélicoïdal (12), **en ce que** le second corps de pièce est une bague (18) solidaire de l'extrémité distale du second ressort hélicoïdal (12), l'ensemble constitué par la bague et le second ressort (12) étant monté en rotation sur le premier corps de pièce (1), **en ce que** la partie distale de la bague (18) est configurée pour venir en butée contre la seconde broche (11), et **en ce que** la partie distale du premier corps de pièce (1) comporte une lumière (6) longitudinale agencée parallèlement au second axe (Y, Y') dans laquelle la seconde broche (11) est configurée pour être insérée.

## Patentansprüche

1. Paar dynamischer Außenbefestigungsvorrichtungen (100) zur Reduzierung von Frakturen von Knochenfragmenten, wobei die Vorrichtungen (100) dazu konfiguriert sind, zu beiden Seiten eines ersten Knochenabschnitts (O1), der mit einem ersten Stift (10) in proximaler Position durchstoßen ist, und eines zweiten Knochenabschnitts (O2), der mit einem zweiten Stift (11) in distaler Position durchstoßen ist, angeordnet zu sein, wobei jede der dynamischen Außenbefestigungsvorrichtungen (100) einen ersten Werkstückkörper (1) und einen zweiten Werkstückkörper umfasst, wobei der erste Werkstückkörper (1) ein längliches Profil aufweist, das sich entlang einer ersten Achse (X, X') erstreckt, die eine proximale Bohrung (5) quer entlang einer zweiten Achse (Y, Y') umfasst, in welcher der erste Stift (10) dazu konfiguriert ist, eingesetzt zu werden, wobei der erste Werkstückkörper (1) in eine erste Schraubenfeder (8) eingebettet ist, **dadurch gekennzeichnet, dass** die erste Schraubenfeder (8) mit einer zweiten Schraubenfeder (12) verwunden ist,
**dadurch gekennzeichnet, dass** der zweite Werkstückkörper ein Ring (18) ist, der fest mit dem distalen Endpunkt der zweiten Schraubenfeder (12) verbunden ist, wobei die durch den Ring und die zweite Feder (12) ausgebildete Einheit drehbar auf dem ersten Werkstückkörper (1) gelagert ist, dass der distale Teil des Rings (18) dazu konfiguriert ist, an den zweiten Stift (11) anzugrenzen, und dass der distale Teil des ersten Werkstückkörpers (1) einen länglichen Hohlraum (6) umfasst, der parallel zur zweiten Achse (Y, Y') angeordnet ist, auf welcher der zweite Stift (11) dazu konfiguriert ist, eingegeben zu werden.

## Claims

1. A pair of dynamic external fixation devices (100) for the reduction of fractures of bone fragments, said devices (100) being configured to be arranged on either side of a first bone portion (O1) through which a first pin (10) passes in the proximal position and of a second bone portion (O2) through which a second pin (11) passes in the distal position, while each of the dynamic external fixation devices (100) comprises a first part body (1) and a second part body, the first part body (1) having a longitudinal profile extending along a first axis (X, X'), including a proximal through-bore (5) along a second axis (Y, Y') into which the first pin (10) is configured to be inserted, said first part body (1) being embedded in a first coil spring (8),
**characterized in that** said first coil spring (8) is nested in a second coil spring (12),
**in that** the second part body is a ring (18) secured to the distal end of the second coil spring (12), the assembly formed by the ring and the second spring (12) being rotatably mounted on the first body part (1), **in that** the distal portion of the ring (18) is configured to come into abutment against the second pin (11),
and **in that** the distal portion of the first part body (1) includes a longitudinal opening (6) arranged parallel to the second axis (Y, Y') into which the second pin (11) is configured to be inserted.
